(19) Europäisches Patentamt
European Patent Office
Office européen des brevets

(11) Veröffentlichungsnummer: **0 073 390**
**B1**

(12) # EUROPÄISCHE PATENTSCHRIFT

(45) Veröffentlichungstag der Patentschrift:
**04.06.86**

(51) Int. Cl.⁴: **A 23 K 1/16,** C 07 D 241/52, C 07 H 17/08

(21) Anmeldenummer: **82107415.0**

(22) Anmeldetag: **16.08.82**

(54) **Wirkstoffkombination aus Kitasamycin und Chinoxalin-di-N-oxiden.**

(30) Priorität: **27.08.81 DE 3133888**

(43) Veröffentlichungstag der Anmeldung:
**09.03.83 Patentblatt 83/10**

(45) Bekanntmachung des Hinweises auf die Patenterteilung:
**04.06.86 Patentblatt 86/23**

(84) Benannte Vertragsstaaten:
**AT BE CH DE FR GB IT LI NL SE**

(56) Entgegenhaltungen:
**EP - A - 0 012 725**
**AT - B - 281 841**
**DE - A - 2 701 707**
**DE - B - 1 670 935**
**US - A - 3 535 309**

(73) Patentinhaber: **BAYER AG, Konzernverwaltung RP Patentabteilung, D-5090 Leverkusen 1 Bayerwerk (DE)**

(72) Erfinder: **Selle, Peter Henry, Dr., 12 Ferry Avenue, Beverley Park New South Wales 2217 (AU)**

**EP 0 073 390 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft antibakteriell wirkende und als Futterzusatzmittel geeignete Wirkstoffkombinationen, bestehend aus Kitasamycin und 2-Methyl-3-carbonsäureamidochinoxalin-1,4-di-N-oxiden, deren Herstellung und ihre Verwendung zur Wachstumsförderung und Verbesserung der Futterverwertung von Tieren.

Es ist bekannt, dass Kitasamycin (s. US-PS 3 535 309; The Merck Index, Ninth Edition) antimicrobiell wirksam ist und auch als Futterzusatzstoff Verwendung findet. Zu der Klasse der 2-Methyl-3-carbonsäureamidochinoxalin-1,4-di-N-oxide gehören sicher die Wirkungsvollsten tierischen Wachstumsförderer (s. DE-PS 1 670 935), so z.B. das N-(2-Hydroxiethyl)-3-methyl-2-chinoxalincarbonsäureamid-1,4-di-N-oxid (Olaquindox).

Es wurde nun gefunden, dass die Kombination von Kitasamycin mit 2-Methyl-3-carbonsäureamdochinoxalin-1,4-di-N-oxiden, insbesondere mit N-(2-Hydroxiethyl)-3-methyl-2-chinoxalin-carbonsäureamid-1,4-di-N-oxid zu einem überadditiven wachstumsfördernden und die Futterverwertung von Tieren verbessernden Effekt führt.

Gegenstand der Erfindung sind daher Wirkstoffkombinationen bestehend aus Kitasamycin und 2-Methyl-3-carbonsäureamidochinoxalin-1,4-di-N-oxiden der allgemeinen Formel I

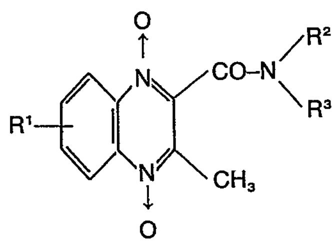

(I)

worin

$R^1$ für Wasserstoff, niedere Alkyl-, niedere Alkoxyreste sowie Chlor steht,

$R^2$ für Wasserstoff, einen gegebenenfalls durch einen Hydroxy-, niederen Alkoxy-. Carbalkoxy-, Mono- oder Dialkylaminorest substituierten, geradkettigen oder verzweigten Alkylrest steht,

$R^3$ für Wasserstoff stehen kann und wobei $R^3$ weiterhin gleich oder verschieden von $R^2$ sein kann und im Falle, dass $R^2$ gleich Wasserstoff ist, $R^3$ auch für den Cyclohexylrest stehen kann, und wobei im Falle, dass $R^2$ und $R^3$ Alkyl bedeuten, diese Reste zusammen mit dem Amidstickstoffatom Bestandteil eines 5- oder 6-gliedrigen heterocyclischen Ringsystems sein können.

Die genannten Alkyl- und Alkoxyreste $R^1$ haben im allgemeinen 1 bis 4 Kohlenstoffatome. Die genannten Alkylreste $R^2$ und $R^3$ enthalten 1 bis 12, vorzugsweise 1 bis 6 Kohlenstoffatome. Die Substituenten (Alkoxy, Carbalkoxy) der genannten Alkylreste $R^2$ und $R^3$ haben im allgemeinen 1 bis 4 Kohlenstoffatome in der Alkylgruppe, die Mono- und Dialkylaminogruppen haben ebenfalls im allgemeinen 1 bis 4 Kohlenstoffatome pro Alkylgruppe. Für den Fall, dass $R^2$ und $R^3$ zusammen Bestandteile eines heterocyclischen Ringsystems sind, kann dieses ausser dem Amidstickstoffatom noch je ein weiteres Stickstoff- oder Sauerstoffatom enthalten, im Falle des 6-Rings befindet sich das weitere Heteroatom bevorzugt in p-Stellung zum Amidstickstoffatom, wobei das Wasserstoffatom am zusätzlichen Stickstoffatom gegebenenfalls durch einen niederen Alkylrest (von $C_1$–$C_4$) substituiert sein kann, der seinerseits wiederum durch eine Hydroxy-, Methoxy- oder Acetoxygruppe substituiert sein kann.

Besonders bevorzugt sind solche Wirkstoffkombinationen, die neben Kitasamycin das N-(2-Hydroxiethyl)-3-methyl-2-chinoxalin-carbonsäureamid-1,4-di-N-oxid enthalten.

Ein weiterer bevorzugter Gegenstand der Erfindung ist die Verwendung der erfindungsgemässen Futterzusatzmittel zur Wachstumsförderung und Verbesserung der Futterverwertung von Tieren.

Bevorzugt enthalten die erfindungsgemässen Kombinationen Kitasamycin und das Chinoxalin-di-N-oxid im Verhältnis 10:1 bis 1:10. Insbesondere wird das Verhältnis 1:1 bevorzugt.

Die Herstellung der Chinoxalin-1,4-di-N-oxide ist ebenfalls wie die des Kitasamycins aus der eingangs erwähnten Literatur bekannt. Unter Kitasamycin wird im erfindungsgemässen Sinn sowohl das Gemisch der biologisch aktiven Komponenten als auch die isolierten einzelnen Komponenten und Gemische zwei oder mehrere der Einzelkomponenten verstanden.

Als Beispiele für die erfindungsgemäss eingesetzten Chinoxalin-di-N-oxide werden die folgenden genannt.

Tabelle 1

| Bsp. Nr. | Formel | F in °C (Z=Zers) | Aussehen |
|---|---|---|---|
| 1 | 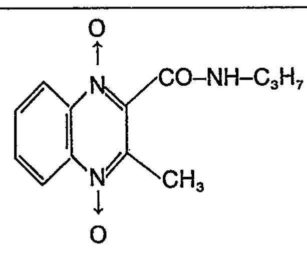 | 172 | hellgelbe Kristalle |

Tabelle 1 (Fortsetzung)

| Bsp. Nr. | Formel | F in °C (Z=Zers) | Aussehen |
|---|---|---|---|
| 2 | 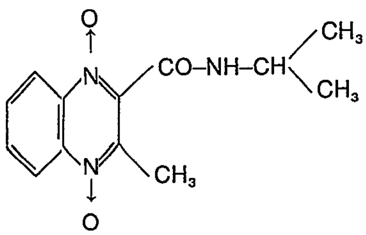 | 208 (Z) | hellgelbe Kristalle |
| 3 | 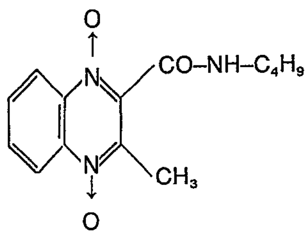 | 136 | dito |
| 4 | 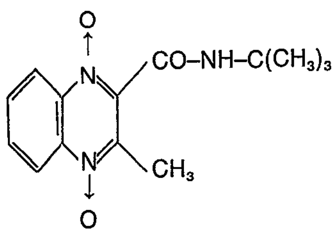 | 214 (Z) | dito |
| 5 | 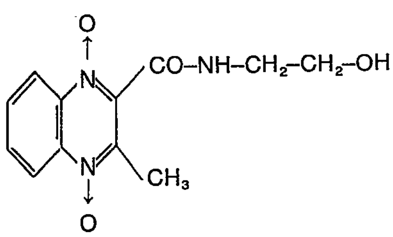 | 209 (Z) | dito |
| 6 | 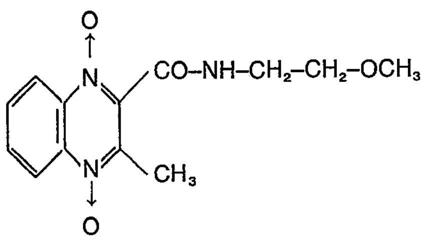 | 135 | dito |
| 7 | 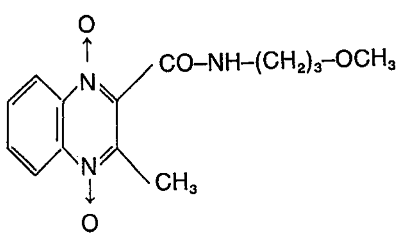 | 141 | dito |
| 8 | 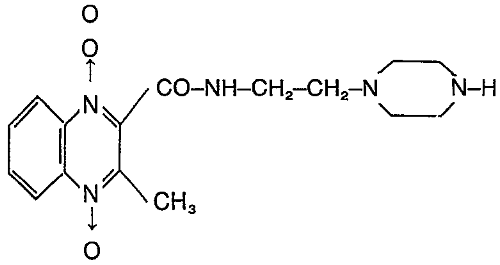 | 218 (Z) | dito |

Tabelle 1 (Fortsetzung)

| Bsp. Nr. | Formel | F in °C (Z=Zers) | Aussehen |
|---|---|---|---|
| 9 | 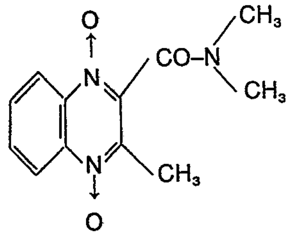 | 189 | hellgelbe Kristalle |
| 10 | 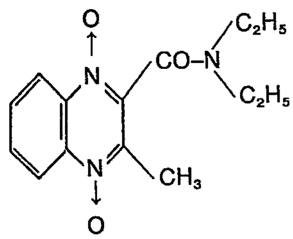 | 162 | dito |
| 11 | 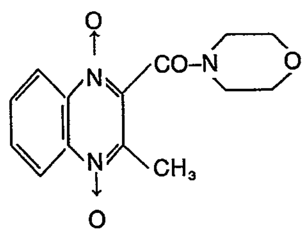 | 231 (Z) | dito |
| 12 | 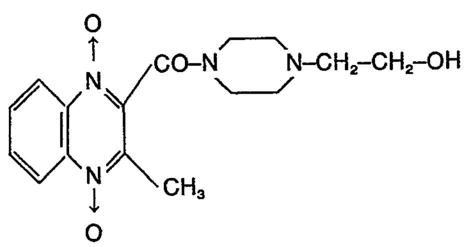 | 203 (Z) | dito |
| 13 | 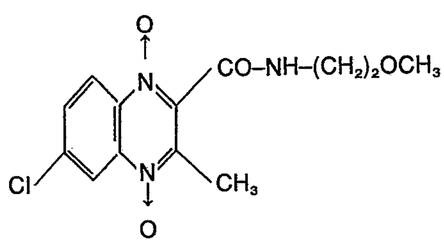 | 183 | gelbe Kristalle |
| 14 | 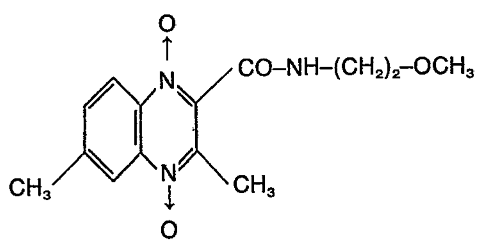 | 169 | rosarote Kristalle |
| 15 | 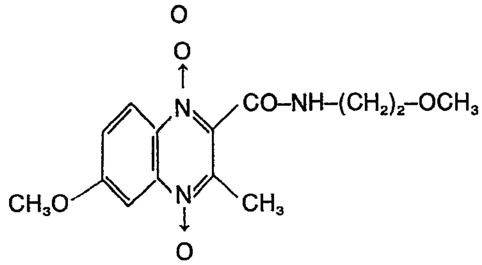 | 190 | gelbe Kristalle |

Tabelle 1 (Fortsetzung)

| Bsp. Nr. | Formel | F in °C (Z=Zers) | Aussehen |
|---|---|---|---|
| 16 | 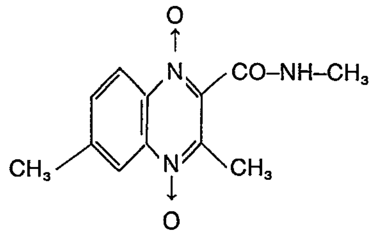 | 202 | gelbe Kristalle |
| 17 | 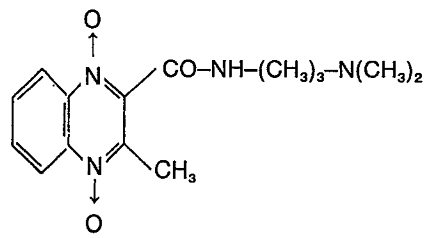 | 154 | gelbe Kristalle |

Die erfindungsgemässen Wirkstoffkombinationen können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der erfindungsgemässen Wirkstoffkombinationen ist hierbei weitgehend unabhängig von der Art und dem Geschlecht der Tiere. Besonders wertvoll erweisen sich die erfindungsgemässen Wirkstoffkombinationen bei der Aufzucht und Haltung von Jung- und Masttieren. Als Tiere, bei denen die erfindungsgemässen Wirkstoffkombinationen zur Förderung und Beschleunigung des Wachstums und zur Verbesserung der Futterverwertung eingesetzt werden können, seien beispielsweise folgende Nutz- und Ziertiere genannt.

Warmblüter wie Rinder, Schweine, Pferde, Schafe, Ziegen, Katzen, Hunde, Kaninchen, Pelztiere, z.B. Nerze und Chinchilla, Geflügel, z.B. Hühner, Gänse, Enten, Truthähne, Tauben, Papageien und Kanarienvögel und Kaltblüter, wie Fische, z.B. Karpfen und Reptilien, z.B. Schlangen.

Die Menge der erfindungsgemässen Wirkstoffkombinationen, die den Tieren zur Erreichung des gewünschten Effektes verabreicht werden, kann wegen der günstigen Eigenschaften der Wirkstoffe weitgehend variiert werden. Sie liegt vorzugsweise bei etwa 5 bis 500, insbesondere 10 bis 100 mg/kg Körpergewicht pro Tag. Die Dauer der Verabreichung kann von wenigen Stunden oder Tagen bis zu mehreren Jahren betragen. Die passende Menge der Wirkstoffe sowie die passende Dauer der Verabreichung hängen insbesondere von der Art, dem Alter, dem Geschlecht, dem Gesundheitszustand und der Art der Haltung der Tiere ab und sind durch jeden Fachmann leicht zu ermitteln.

Die erfindungsgemässen Wirkstoffkombinationen werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmässigen oder unregelmässigen Abständen oral oder parenteral erfolgen. Aus Zweckmässigkeitsgründen ist in den meisten Fällen eine orale Verabreichung, insbesondere im Thytmus der Nahrungs- und/oder Getränkeaufnahme der Tiere, vorzuziehen.

Die erfindungsgemässen Wirkstoffkombinationen können als reine Stoffmischung oder in formulierter Form, also in Mischung mit nichttoxischen inerten Trägerstoffen beliebiger Art, z.B. mit Trägerstoffen und in Formulierungen, wie sie bei nutritiven Zubereitungen üblich sind, verabreicht werden.

Die erfindungsgemässen Wirkstoffkombinationen werden gegebenenfalls in formulierter Form zusammen mit pharmazeutischen Wirkstoffen, Mineralsalzen, Spurenelementen, Vitaminen, Eiweissstoffen, Fetten, Farbstoffen und/oder Geschmackstoffen in geeigneter Form verabreicht.

Empfehlenswert ist die orale Verabreichung zusammen mit dem Futter und/oder Trinkwasser, wobei je nach Bedarf die Wirkstoffkombinationen der Gesamtmenge oder nur Teilen des Futters und/oder des Trinkwassers zugegeben werden.

Die erfindungsgemässen Wirkstoffkombinationen werden nach üblichen Methoden durch einfaches Mischen als reine Stoffmischung, vorzugsweise in fein verteilter Form oder in formulierter Form in Mischung mit essbaren nichttoxischen Trägerstoffen, gegebenenfalls in Form eines Praemix oder eines Futterkonzentrates, dem Futter und/oder dem Trinkwasser beigefügt.

Das Futter und/oder das Trinkwasser kann beispielsweise die erfindungsgemässen Wirkstoffkombinationen in einer Gewichtskonzentration von etwa 5 bis 500, insbesondere 10 bis 100 ppm enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschliesslich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die erfindungsgemässen Wirkstoffkombinationen neben essbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten kann die Wirkstoffkombination gegebenenfalls auch durch ihre Oberfläche bedeckende geeignete Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das eine erfindungsgemässe Wirkstoffkombination enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

Die Vitamin-Mineral-Mischung besteht aus:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg $Mn\ SO_4 \times H_2O$, 140 mg $Zn\ SO_4 \times 7\ H_2O$, 100 mg $FE\ SO_4 \times 7\ H_2O$ und 20 mg $Cu\ SO_4 \times 5\ H_2O$.

Der Wirkstoff-Praemix enthält die erfindungsgemässen Wirkstoffkombinationen in der gewünschten Menge, z.B. 100 mg und zusätzlich 1 g DL-Methionin sowie so viel Sojabohnenmehl, dass 2,5 g Praemix entstehen.

Beispiel für die Zusammensetzung eines Schweineaufzuchtfutters, das die erfindungsgemässe Wirkstoffkombination enthält:

630 g Futtergetreideschrot (zusammengesetzt aus 200 g Mais, 150 g Gerste-, 150 g Hafer und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Mit den erfindungsgemässen Wirkstoffkombinationen wurden mehrere Fütterungsversuche durchgeführt.

Dabei wurden folgende Ergebnisse erhalten.

Beispiel 1

| a) | Tier-Charakteristik und Futter | |
|---|---|---|
| a1) | Ferkel | |
| a2) | Anzahl | 30 |
| a3) | Zucht | grosse weisse Landrasse Kreuzungszucht |
| a4) | Geschlecht | männlich |
| a5) | Alter | etwa 8 bis 14 Wochen |
| a6) | Gewicht | 17,5 bis 45 kg |
| a7) | Zustand | gut |
| a8) | Futter | |
| | Rohprotein (mind.) | 17,0% |
| | Rohfett | 2,5% |
| | Faseranteil | 5,0% |
| | Salz | 0,5% |
| | Harnstoff (Max) | 0,0% |
| | Fluor (Max) | 0,01% |
| | Lysin | 0,832% |
| | Methionin | 0,249% |

Beispiel 1 (Fortsetzung)

| a) | Tier-Charakteristik und Futter | |
|---|---|---|
| | Threonin | 0,494% |
| | Isoleucin | 0,564% |
| | Methionin + Cystein | 0,522% |
| | Thryptophan | 0,209% |
| | Biotin | 0,108% |
| | Cholin | 0,110% |
| | Calcium | 1,035% |
| | Phosphor | 0,790% |

Ingredientien:

Weizen, Gerste, Hirse, Kleie, Fleischmehl, Sojabohnenmehl, Baumwollsamenmehl, Sonnenblumenmehl, Lysin, Salz, Kalkstein, 2RO, MnO, Kupfer- und Eisensulfat, Kaliumjodid, Vitamine A, D, E, K, B/2, Niazin, Pantothensäure.

b) Behandlung der Tiere

Die Behandlung der Tiere wurde in 5 Behandlungsgruppen durchgeführt. Das Anfangsgewicht für jede Gruppe war gleich.

| | | ppm |
|---|---|---|
| b1) | Olaquindox | 50 |
| b2) | Kitasamycin | 50 |
| b3) | Olaquindox + Kitasamycin | 25:25 |
| b4) | Olaquindox + Kitasamycin | 12,5:12,5 |
| b5) | Kontrolle (ohne Wirkstoffe) | – |

c) Auswertung

Die Ferkel wurden täglich gewogen. Es wurde die tägliche Gewichtszunahme und Futterverwertung bestimmt. Wöchentliches Wiegen und individuelle Futteraufnahme wurden notiert.

| d) | Ergebnis (Gewichtszunahme g/Tag)<br>Konzentration (ppm) | Durchschnittliche tägliche Gewichtszunahme (g/Tag) |
|---|---|---|
| d1) | 50 | 593 |
| d2) | 50 | 562 |
| d3) | 25:25 | 626 |
| d4) | 12,5:12,5 | 560 |
| d5) | Kontrolle | 537 |

Der synergistische Effekt bei der Gewichtszunahme ist signifikant.

| e) | Ergebnis (Futterverwertung)<br>Konzentration (ppm) | Durchschnittliches Futterverwertungsverhältnis (g/g) |
|---|---|---|
| e1) | | 2,52 |
| e2) | | 2,64 |
| e3) | | 2,44 |
| e4) | | 2,66 |
| e5) | | 2,72 |

Der synergistische Effekt bei der Futterverwaltung ist signifikant.

Beispiel 2

| a) | Tiercharakteristik und Futter | |
|---|---|---|
| a1) | Ferkel | |
| a2) | Anzahl | 84 |
| a3) | Geschlecht | gemischt |

Beispiel 2 (Fortsetzung)

| a) | Tiercharakteristik und Futter | |
|---|---|---|
| a4) | Zucht | grosse weisse Landrasse Kreuzungszucht |
| a5) | Alter | 5 Wochen |
| a6) | Gewicht | 7 bis 75 kg |
| a7) | Zustand | gut |
| a8) | Futter | |

| 18% Rohprotein Starter | kg/Tonne |
|---|---|
| Weizen | 730 |
| Fleischmehl | 100 |
| Sojamehl | 120 |
| Sonnenblumenmehl | 30 |
| Mineralien- u. Vitamine-Prämix | 10 |
| Pflanzl. Öl | 10 |

| 16% Rohprotein Finisher | |
|---|---|
| Weizen | 804 |
| Fleischmehl | 125 |
| Sonnenblumenmehl | 60 |
| Mineralien u. Vitamin-Mischung | 10 |
| Methionin | 1 |

| b) | Behandlung der Tiere | Dosierung ppm bis zu 40 kg | bis zu 75 kg |
|---|---|---|---|
| b1) | Olaquindox | 100 | 25 |
| b2) | Kitasamycin | 55 | 11 |
| b3) | Olaquindox+ | 25 | 25 |
| | Kitasamycin | 22 | 11 |
| b4) | Kontrolle | – | – |

c) Auswertung

Die Ferkel wurden zu Versuchsbeginn und in wöchentlichen oder vierzehntägigen Abständen bis zum Versuchsende gewogen.

Futtermenge und Mortalität wurden täglich notiert.

Die Rückenfettstärke wurde per Ultraschall am Versuchsende gemessen und auf 75 kg Lebendgewicht korrigiert.

| d) | Ergebnis (Gewichtszunahme g/Tag) Beginn bis 40 kg | 40 kg bis Ende | Beginn bis Ende |
|---|---|---|---|
| d1) | 499 | 641 | 573 |
| d2) | 508 | 574 | 522 |
| d3) | 517 | 624 | 568 |
| d4) | 460 | 593 | 531 |

| e) | Ergebnis (Futterverwertung g/g) Beginn bis 40 kg | 40 kg bis 75 kg | Beginn bis Ende |
|---|---|---|---|
| e1) | 2,30 | 3,10 | 2,74 |
| e2) | 2,28 | 3,17 | 2,74 |
| e3) | 2,15 | 2,80 | 2,48 |
| e4) | 2,41 | 3,18 | 2,81 |

| f) | Ergebnis (Rückenfettstärke) Rückenfett (mm) |
|---|---|
| f1) | 21,3 |
| f2) | 20,3 |
| f3) | 20,7 |
| f4) | 21,6 |

Auch im Beispiel 2 lässt sich die Überlegenheit des Kombinationspräparates deutlich erkennen.

**Patentansprüche**

1. Wirkstoffkombination bestehend aus Kitasamycin und 2-Methyl-3-carbonsäureamidochinoxalin-1,4-di-N-oxiden der allgemeinen Formel I

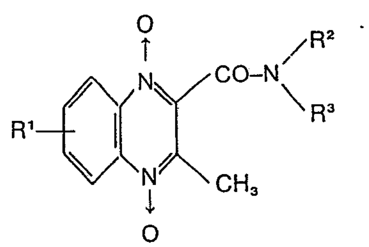

(I)

worin

$R^1$ für Wasserstoff, niedere Alkyl-, niedere Alkoxyreste sowie Chlor steht,

$R^2$ für Wasserstoff, einen gegebenenfalls durch einen Hydroxy-, niederen Alkoxy-, Carbalkoxy-, Mono- oder Dialkylaminorest substituierten, geradkettigen oder verzweigten Alkylrest steht,

$R^3$ für Wasserstoff stehen kann und wobei $R^3$ weiterhin gleich oder verschieden von $R^2$ sein kann und im Falle, dass $R^2$ gleich Wasserstoff ist, $R^3$ auch für den Cyclohexylrest stehen kann, und wobei im Falle, dass $R^2$ und $R^3$ Alkyl bedeuten, diese Reste zusammen mit dem Amidstickstoffatom Bestandteil eines 5- oder 6-gliedrigen heterocyclischen Ringsystems sein können.

2. Wirkstoffkombination nach Anspruch 1, bestehend aus Kitasamycin und N-(2-Hydroxiethyl)-3-methyl-2-chinoxalincarbonsäureamid-1,4-di-N-oxid.

3. Wirkstoffkombination nach Anspruch 1, enthaltend Kitasamycin und Verbindungen der Formel I im Verhältnis 1:10 bis 10:1.

4. Wirkstoffkombination nach Anspruch 1, enthaltend Kitasamycin und Verbindungen der Formel I im Verhältnis 1:1.

5. Futterzusatzmittel, enthaltend die Wirkstoffkombination nach Anspruch 1.

6. Futterzusatzmittel, enthaltend die Wirkstoffkombination nach Anspruch 2.

7. Futterzusatzmittel, enthaltend die Wirkstoffkombination nach Anspruch 2 im Verhältnis 1:10 bis 10:1.

8. Futterzusatzmittel, enthaltend die Wirkstoffkombination nach Anspruch 2 im Verhältnis 1:1.

9. Prämixe für die Futterherstellung, enthaltend die Wirkstoffkombination nach Anspruch 1.

10. Verwendung von Kitasamycin in Kombination mit Verbindungen der Formel I nach Anspruch 1 in Futterzusatzmitteln und Prämixen für die Futterherstellung.

**Claims**

1. Active compound combination consisting of kitasamycin and 2-methyl-3-carboxamidoquinoxaline-1,4-di-N-oxides of the general formula I

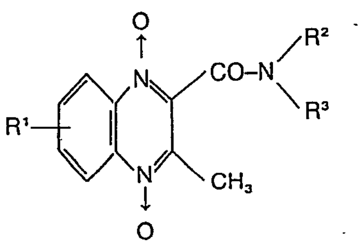

(I)

wherein

$R^1$ represents hydrogen, lower alkyl, lower alkoxy radicals and chlorine,

$R^2$ represents hydrogen or a straight-chain or branched alkyl radical which is optionally substituted by a hydroxyl, lower alkoxy, carboalkoxy, mono- or dialkylamino radical,

$R^3$ can represent hydrogen and wherein $R^3$ furthermore can be identical to or different from $R^2$ and in the case where $R^2$ is hydrogen $R^3$ can also represent the cyclohexyl radical and wherein in

the case where $R^2$ and $R^3$ denote alkyl these radicals, together with the amide nitrogen atom, can be a constituent of a 5- or 6-membered heterocyclic ring system.

2. Active compound combination according to Claim 1, consisting of kitasamycin and N-(2-hydroxyethyl)-3-methyl-2-quinoxalinecarboxamide-1,4-di-N-oxide.

3. Active compound combination according to Claim 1, containing kitasamycin and compounds of the formula I in a ratio of 1:10 to 10:1.

4. Active compound combination according to Claim 1, containing kitasamycin and compounds of the formula I in a ratio of 1:1.

5. Feed additive containing the active compound combination according to Claim 1.

6. Feed additive containing the active compound combination according to Claim 2.

7. Feed additive containing the active compound combination according to Claim 2 in a ratio of 1:10 to 10:1.

8. Feed additive containing the active compound combination according to Claim 2 in a ratio of 1:1.

9. Premixes for the production of feeds, containing the active compound combination according to Claim 1.

10. Use of kitasamycin in combination with compounds of the formula I according to Claim 1 in feed additives and premixes for the production of feeds.

**Revendications**

1. Association de substances actives formée de kitasamycine et de 1,4-di-N-oxydes de 2-méthyl-3-carboxamidoquinoxaline, de formule générale I

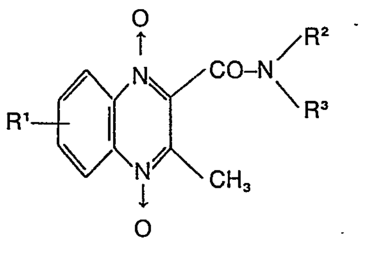

(I)

dans laquelle

$R^1$ représente l'hydrogène, des restes alkyle inférieur, alkoxy inférieur ainsi que du chlore,

$R^2$ désigne l'hydrogène, un reste alkyle à chaîne droite ou à chaîne ramifiée éventuellement substitué par un reste hydroxy, alkoxy inférieur, carbalkoxy, monoalkylamino ou dialkylamino,

$R^3$ peut représenter l'hydrogène et $R^3$ peut en outre être identique à $R^2$ ou en être différent et au cas où $R^2$ représente l'hydrogène, $R^3$ peut aussi représenter le reste cyclohexyle, et au cas où $R^2$ et $R^3$ désignent des restes alkyle, ces restes peuvent former conjointement avec l'atome d'azote d'amide un composant d'un système de noyau hétérocyclique pentagonal ou hexagonal.

2. Association de substances actives suivant la revendication 1, composée de kitasamycine et de 1,4-di-N-oxyde de N-(2-hydroxyéthyl)-3-méthyl-2-quinoxalinecarboxamide.

3. Association de substances actives suivant la revendication 1, contenant de la kitasamycine et des composés de formule I dans un rapport de 1:10 à 10:1.

4. Association de substances actives suivant la revendication 1, contenant de la kitasamycine et des composés de formule I dans un rapport de 1:1.

5. Additif pour aliments, contenant l'association de substances actives suivant la revendication 1.

6. Additif pour aliments contenant l'association de substances actives suivant la revendication 2.

7. Additif pour aliments contenant l'association de substances actives suivant la revendication 2 dans un rapport de 1:10 à 10:1.

8. Additif pour aliments contenant l'association de substances actives suivant la revendication 2 dans le rapport de 1:1.

9. Mélanges préalables pour la préparation d'aliments, contenant l'association de substances actives suivant la revendication 1.

10. Utilisation de kitasamycine en association avec des substances actives de formule I suivant la revendication 1 dans des mélanges préalables pour la préparation d'aliments.